# EUROPEAN PATENT APPLICATION

(11) **EP 2 452 657 A1**
(43) Date of publication of application: **16.05.2012**
(21) Application number: 11008909.1
(22) Date of filing: 09.11.2011
(51) Int. Cl.: A61F 11/00, A61B 5/12

(54) **A mobile hearing treatment equipment**

(30) Priority: 11.11.2010 CN 201010540218
(71) Applicant: Eartronic Ltd., Mahe (SC)
(72) Inventor: Fei Shi, Meng, Lu Wan District, Shanghai 200020 (CN)
(74) Representative: Ostermann, Thomas

(57) **Abstract**

The invention relates to a mobile hearing treatment equipment which comprises a central processing unit, a first interface unit, an audio decoder, a digital/analog converter, an amplifier and a loudspeaker. The mobile hearing treatment equipment is connected with a hearing test equipment and can download audio files for treatment. An intelligent chip is adopted so as to make the hearing treatment equipment compact and easy to carry. The intelligent chip can intelligently control the sound output so that user can easily operate the equipment. The audio file for treatment can be updated from the hearing test equipment through the first interface unit whenever necessary.

## Description

### Field of technology

The invention relates to the medical appliance field, in particular to a mobile hearing treatment equipment.

### Background art

Except for drug therapy, external stimulation therapy can also be used for hearing treatment. The patent US2009/0149916A1 discloses a method and an equipment utilizing acoustic signal to stimulate hair cells. Many patients have been cured since this kind of equipment was put into service. However, hearing impaired patient has to receive treatment at a doctor's surgery until today and the treatment lasts 30-120min each time and has to be repeatedly performed many times. Normally this therapy lasts 2-8 weeks during which treatment shall be performed everyday. Being treated everyday in hospital brings great inconvenience to both the patient and the hospital. Moreover, the patient can not select time and place for treatment by himself/herself.

### Content of the invention

To solve abovementioned problem, the invention provides a mobile hearing treatment equipment which can be easily operated and has excellent treatment effect:
For this reason, the invention adopts the following technical solution: a mobile hearing treatment equipment is characterized in that it comprises a central processing unit, a first interface unit, an audio decoder, a digital/analog converter, an amplifier and a loudspeaker.
The mobile hearing treatment equipment is connected with a hearing test equipment and can download audio files for treatment;
The first interface unit is connected with the hearing test equipment and the central processing unit for downloading the audio file for treatment and transmitting the file to central processing unit;
The central processing unit receives the audio file for treatment and controls the decoding of the audio file for treatment, digital/analog converting and amplification; The audio decoder is connected with the central processing unit and the digital/analog converter and used for decoding the audio file for treatment into digital audio signals for treatment and sending the signals to digital/analog converter;
The digital/analog converter is connected with the central processing unit and used for converting the digital audio signals for treatment into analog audio signals for treatment and sending the signals to the amplifier;
The amplifier is connected with the central processing unit and the loudspeaker and used for amplifying the analog audio signals for treatment and transmitting the signals to the loudspeaker;

The loudspeaker is used for emitting sound waves for treatment.
The technical solution adopted in this invention is further optimized that a hearing test equipment which is connected with the mobile hearing treatment equipment comprises a control unit, a hearing test unit, a sound generating unit, a sound storage unit and a second interface unit;
The control unit is used for configuring and controlling the hearing test unit, the sound generating unit, the sound storage unit and the second interface unit;
The hearing test unit is connected with the control unit and used for testing user's hearing as well as generating treatment audio-frequency according to the test result and then transmitting it to the control unit;
The sound generating unit is connected with the control unit and used for receiving the treatment audio-frequency as well as generating the audio file for treatment according to the treatment audio-frequency and then transmitting the audio file for treatment to the control unit;
The sound storage unit is connected with the control unit and used for receiving and storing the audio file for treatment;

The second interface unit is connected with the control unit and used for connecting with the first interface unit on the mobile hearing treatment equipment for downloading and sending the audio file for treatment to the mobile hearing treatment equipment.

The technical solution adopted in this invention is further optimized that it also comprises a sound volume adjusting unit which is connected with the central processing unit and used for inputting user's control information.

The technical solution adopted in this invention is further optimized that it also comprises a display unit used for displaying the information about sound playing. The technical solution adopted in this invention is further optimized that it also comprises an audio file memory unit which is connected with the central processing unit and used for storing the audio file for treatment. The technical solution adopted in this invention is to use the hearing test equipment to test the hearing and therefore generate audio signals for treatment which is saved as an audio file for treatment. The mobile hearing treatment equipment is used for playing the audio file for treatment to reach the purpose of treating and improving the hearing.

The invention has the advantage that an intelligent chip is adopted so as to make the hearing treatment equipment compact and easy to carry. The intelligent chip can intelligently control the sound output so that user can easily operate the equipment. The audio file for treatment can be updated from the hearing test equipment through the first interface unit whenever necessary.

The invention will be described in details hereinafter through combining the embodiments and the diagrams.

### Diagrams description

- Figure 1: is the schematic diagram of circuit module of this embodiment.
- Figure 2: is the schematic diagram of software module of this embodiment.
- Figure 3: is the schematic diagram of hearing test equipment of this embodiment.
- Figure 4: is the workflow diagram of this embodiment.

### Detailed implementation method

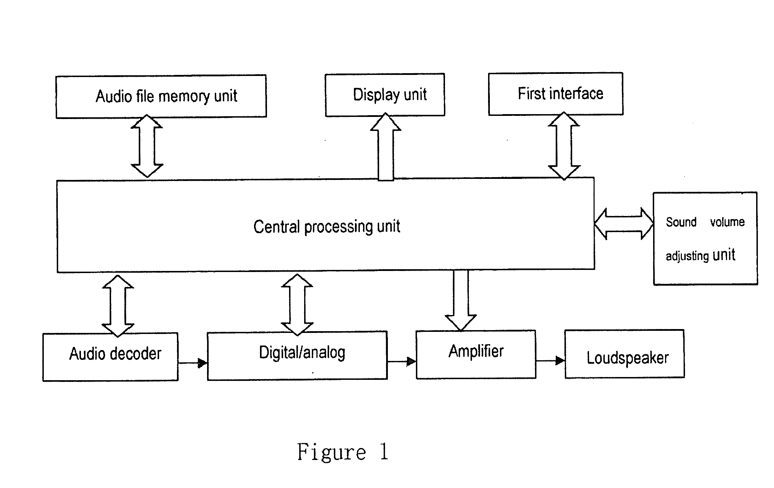

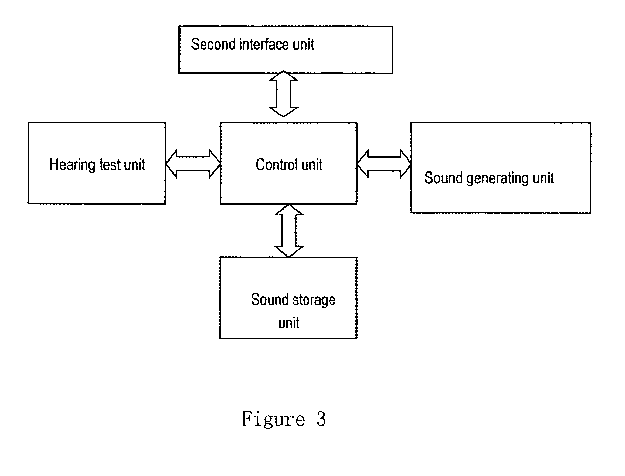

Referring to Figure 1 & Figure 3, a mobile hearing treatment equipment comprises a central processing unit, a first interface unit, an audio decoder, a digital/analog converter, an amplifier and a loudspeaker. The mobile hearing treatment equipment is connected with a hearing test equipment and can download audio files for treatment; The first interface unit is connected with the hearing test equipment and the central processing unit for downloading the audio file for treatment and transmitting the file to central processing unit;
The central processing unit receives the audio file for treatment and controls the decoding of the audio file for treatment, digital/analog converting and amplification; The audio decoder is connected with the central processing unit and the digital/analog converter and used for decoding the audio file for treatment into digital audio signals for treatment and sending the signals to digital/analog converter;
The digital/analog converter is connected with the central processing unit and used for converting the digital audio signals for treatment into analog audio signals for treatment and sending the signals to the amplifier;
The amplifier is connected with the central processing unit and the loudspeaker and used for amplifying the analog audio signals for treatment and transmitting the signals to the loudspeaker;

The loudspeaker is used for emitting sound waves for treatment.
A hearing test equipment which is connected with the mobile hearing treatment equipment comprises a control unit, a hearing test unit, a sound generating unit, a sound storage unit and a second interface unit;
The control unit is used for configuring and controlling the hearing test unit, the sound generating unit, the sound storage unit and the second interface unit;
The hearing test unit is connected with the control unit and used for testing user's hearing as well as generating treatment audio-frequency according to the test result and then transmitting it to the control unit;
The sound generating unit is connected with the control unit and used for receiving the treatment audio-frequency as well as generating the audio file for treatment according to the treatment audio-frequency and then transmitting the audio file for treatment to the control unit;
The sound storage unit is connected with the control unit and used for receiving and storing the audio file for treatment;

The second interface unit is connected with the control unit and used for connecting with the first interface unit on the mobile hearing treatment equipment for downloading and sending the audio file for treatment to the mobile hearing treatment equipment.

It also comprises a sound volume adjusting unit which is connected with the central processing unit and used for inputting user's control information. So the equipment has a better interactivity and user can adjust sound volume or switch it on/off opportunely.

It also comprises a display unit used for displaying the information about sound playing. So the equipment can be easily controlled and has a better interactivity and user can know the sound information more intuitively.

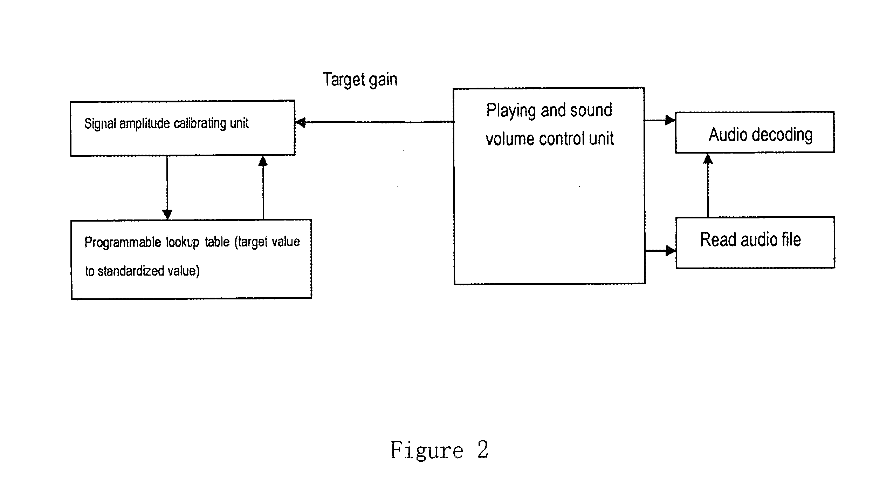

It also comprises an audio file memory unit which is connected with the central processing unit and used for storing the audio file for treatment. Bigger memory space makes the application range of the equipment wider. Referring to Figures 1 & 2, the mobile hearing treatment equipment is arranged in both ears. The central processing unit (intelligent chip) converts the audio file for treatment stored in audio file memory unit into standard analog treatment sound signals which is subsequently transformed into sound waves by the loudspeaker. Treatment sound wave with specific frequency is played to stimulate specific hair cells in order to treat and improve the hearing of patient. Through the first interface unit, the central processing unit saves the audio file for treatment into the audio file memory unit. Once the patient starts up the system, the central processing unit reads the audio file for treatment, format of which can be WAVE, MP3 or WMA, from the audio file memory unit. If the audio file for treatment is compressed, the central processing unit transmits the audio file for treatment into audio decoder for decoding and the obtained uncompressed audio files after decoding are digital. The digital audio treatment signals are transmitted by the central processing unit into the digital/analog converter for converting after which the digital audio treatment signals are converted into analog treatment signals. Use the amplifier to amplify the analog treatment signals till specified gain is obtained. Finally the signals are transmitted into patient's ears through left and right loudspeakers. Referring to Figures 1 & 2, when listening to treatment music, the patient can use the sound volume adjusting unit to adjust the sound volume himself/herself. Once receiving the signal for increasing or decreasing volume, the central processing unit controls the amplifier to adjust the volume correspondingly. Because the audio file for treatment is generated in the hearing test equipment but played in the mobile treatment equipment, the mobile hearing treatment equipment has to be calibrated in order to ensure same sound amplitude for both equipments. This is realized through the signal amplitude calibration unit shown in Figure 2. For example, if the patient can hear the sound with intensity less than 50dB at a specific frequency in the hearing test equipment, the sound is downloaded into the mobile hearing treatment equipment and then use the sound volume adjusting unit to adjust volume to the lowest level the patient can hear.At this time record the sound wave output signals from the mobile hearing treatment equipment and therefore generate a form which is stored in the audio file memory unit.

Referring to Figure 3, the control unit is used for configuring and controlling the other units and the hearing test unit is used for examining the ear hearing and distinguishing the frequency at which the patient can be treated. The sound generating unit generates the audio file for treatment with specific frequency and intensity according to patient's frequency and stores it into the sound storage unit. Wherein the sound generating program is based on the hair cell stimulation method (see patent US2009/0149916A1) and uses predetermined algorithm to calculate the bandwidth which corresponds to the hair cell impaired area through and is taken as the target bandwidth. Then output audio signals with given intensity to stimulate the hair cell impaired area.

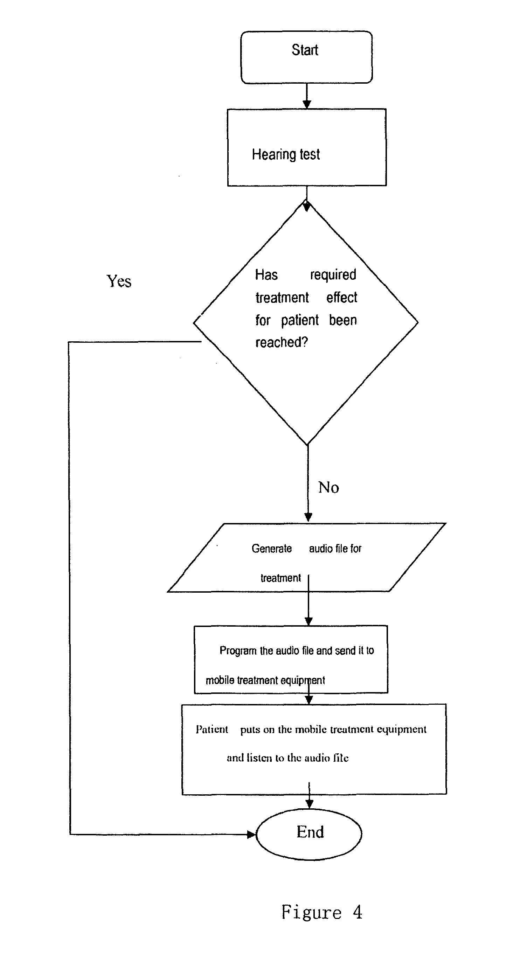

Referring to Figure 4, firstly patient goes to hospital and the doctor uses the hearing test equipment to examine ears and generate a test report. If patient can not clearly hear sound at some specific frequencies, the doctor generates specific audio files for treatment according to these specific frequencies. Then connect the hearing test equipment with the mobile hearing treatment equipment through interfaces. So the patient can use the mobile hearing treatment equipment to listen to the audio file for treatment. After a period of time, the patient goes to hospital for reexamination and the doctor can reexamine the hearing and generate new audio file for treatment according to patient treatment. Repeat above process for patient until the doctor thinks hearing is obviously improved or stops it. The patient can adjust the music volume himself/herself during listening.

Note for technical staff: the solution adopted in this invention is described through above embodiment but not limited to it.

## Claims

1. A mobile hearing treatment equipment is **characterized in that** it comprises a central processing unit, a first interface unit, an audio decoder, a digital/analog converter, an amplifier and a loudspeaker. The mobile hearing treatment equipment is connected with a hearing test equipment and can download audio files for treatment; The first interface unit is connected with the hearing test equipment and the central processing unit for downloading the audio file for treatment and transmitting the file to central processing unit;
The central processing unit receives the audio file for treatment and controls the decoding of the audio file for treatment, digital/analog converting and amplification; The audio decoder is connected with the central processing unit and the digital/analog converter and used for decoding the audio file for treatment into digital audio signals for treatment and sending the signals to digital/analog converter;
The digital/analog converter is connected with the central processing unit and used for converting the digital audio signals for treatment into analog audio signals for treatment and sending the signals to the amplifier;
The amplifier is connected with the central processing unit and the loudspeaker and used for amplifying the analog audio signals for treatment and transmitting the signals to the loudspeaker;
The loudspeaker is used for emitting sound waves for treatment.

2. A mobile hearing treatment equipment as claimed in Claim 1 is **characterized in that** a hearing test equipment which is connected with the mobile hearing treatment equipment comprises a control unit, a hearing test unit, a sound generating unit, a sound storage unit and a second interface unit.
The control unit is used for configuring and controlling the hearing test unit, the sound generating unit, the sound storage unit and the second interface unit;
The hearing test unit is connected with the control unit and used for testing user's hearing as well as generating treatment audio-frequency according to the test result and then transmitting it to the control unit;
The sound generating unit is connected with the control unit and used for receiving the treatment audio-frequency as well as generating the audio file for treatment according to the treatment audio-frequency and then transmitting the audio file for treatment to the control unit;
The sound storage unit is connected with the control unit and used for receiving and storing the audio file for treatment;
The second interface unit is connected with the control unit and used for connecting with the first interface unit on the mobile hearing treatment equipment for downloading and sending the audio file for treatment to the mobile hearing treatment equipment.

3. A mobile hearing treatment equipment as claimed in Claim 1 is **characterized in that** it also comprises a sound volume adjusting unit which is connected with the central processing unit and used for inputting user's control information.

4. A mobile hearing treatment equipment as claimed in Claim 1 or 3 is **characterized in that** it also comprises a display unit used for displaying the information about sound playing.

5. A mobile hearing treatment equipment as claimed in Claim 1 is **characterized in that** it also comprises an audio file memory unit which is connected with the central processing unit and used for storing the audio file for treatment.
